(19) Europäisches Patentamt
European Patent Office
Office européen des brevets

(11) Veröffentlichungsnummer: **0 008 453**
**B1**

(12) # EUROPÄISCHE PATENTSCHRIFT

(45) Veröffentlichungstag der Patentschrift: **29.04.81**

(21) Anmeldenummer: **79103045.5**

(22) Anmeldetag: **20.08.79**

(51) Int. Cl.³: **C 07 C 17/32,**
C 07 C 43/225,
C 07 C 43/29,
C 07 C 149/32

(54) **Verfahren zur Herstellung aromatischen Trifluormethylverbindungen.**

(30) Priorität: **28.08.78 DE 2837499**

(43) Veröffentlichungstag der Anmeldung:
**05.03.80 Patentblatt 80/5**

(45) Bekanntmachung des Hinweises auf die Patenterteilung:
**29.04.81 Patentblatt 81/17**

(84) Benannte Vertragsstaaten:
**BE CH DE FR GB IT NL**

(56) Entgegenhaltungen:
**FR - A - 1 602 719**
**GB - A - 775 581**
**GB - A - 1 180 400**

(73) Patentinhaber: **BAYER AG**
**Zentralbereich Patente, Marken und Lizenzen**
**D-5090 Leverkusen 1, Bayerwerk (DE)**

(72) Erfinder: **Marhold, Albrecht, Dr.**
**Carl-Duisbergstrasse 329**
**D-5090 Leverkusen (DE)**
Erfinder: **Klauke, Erich, Dr.**
**Eichendorffweg 8**
**D-5068 Odenthal (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 008 453 B1

## Verfahren zur Herstellung von aromatischen Trifluormethylverbindungen

Die Erfindung betrifft ein Verfahren zur Herstellung von aromatischen Trifluormethylverbindungen.

Es ist bekannt, Benzotrifluorid durch Fluorierung von Benzotrichlorid mit wasserfreier Flußsäure herzustellen (Houben-Weyl, Methoden der organischen Chemie, Band V/3, Seite 121, Georg Thieme, Stuttgart 1962). Dieses Verfahren ist auf Verbindungen beschränkt, die keine weiteren Substituenten tragen, die bei der Chlorierung einer Methylgruppe ebenfalls chloriert werden. So ist beispielsweise 4-Methyl-benzotrichlorid nach dem genannten Verfahren nicht zugänglich. Außerdem ist dieses Verfahren stets zweistufig, da zuerst eine Methylgruppe zur Trichlormethylgruppe chloriert werden muß und erst dann durch Fluorierung das gewünschte Benzotrifluorid hergestellt werden kann.

Es wurde ein Verfahren zur Herstellung von aromatischen Trifluormethylverbindungen gefunden, das dadurch gekennzeichnet ist, daß man eine aromatische Verbindung der Formel

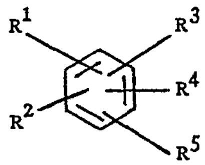

(I)

in der

$R^1$ Wasserstoff, Alkyl, Aryl, Aralkyl, Aryloxy, Arylthio, Polyhalogenalkyloxy oder Polyhalogenalkylthio bedeutet, wobei die aromatischen Substituenten $R^1$ ihrerseits durch Halogen, Alkyl, Polyhalogenalkyl, Polyhalogenalkyloxy, Polyhalogenalkylthio, Nitro, Chlorcarbonyl oder Chlorsulfonyl substituiert sein können,

$R^2$, $R^3$, $R^4$ und $R^5$ gleich oder verschieden sind und Wasserstoff, Halogen oder Alkyl bedeuten,

sowie zwei benachbarte Reste aus der Gruppe $R^1$ bis $R^5$ gemeinsam einen drei- bis fünf-gliedrigen Alkylenrest bilden können,

wobei die Substituenten $R^1$ bis $R^5$, wenn sie Alkyl bedeuten, durch Halogenalkyl substituiert sein können, mit Tetrachlorkohlenstoff und Fluorwasserstoff im Temperaturbereich von 50 bis 140°C umsetzt.

Halogensubstituenten ($R^2$ bis $R^5$) sind Fluor, Chlor oder Brom.

Alkylsubstituenten ($R^1$ bis $R^5$) können solche mit 1 bis 8 Kohlenstoffatomen sein, beispielsweise Methyl, Äthyl, Propyl, Butyl, Hexyl oder Octyl, bevorzugt solche mit 1 bis 4 Kohlenstoffatomen, ganz bevorzugt Methyl.

Arylsubstituenten ($R^1$) können solche mit 6 bis 12 Kohlenstoffatomen sein, beispielsweise Phenyl oder Diphenyl, bevorzugt Phenyl.

Aralkylsubstituenten ($R^1$) können solche mit 1 oder 2 Kohlenstoffatomen im aliphatischen Teil und 6 bis 12 Kohlenstoffatomen im aromatischen Teil sein, beispielsweise Benzyl, Phenyläthyl, Diphenylmethyl oder Diphenyläthyl, bevorzugt Benzyl.

Aryloxysubstituenten ($R^1$) können solche mit 6 bis 12 Kohlenstoffatomen sein, beispielsweise Phenyloxy oder Diphenyloxy, bevorzugt Phenyloxy.

Arylthio-Substituenten ($R^1$) können solche mit 6 bis 12 Kohlenstoffatomen sein, beispielsweise Phenylthio oder Diphenylthio, bevorzugt Phenylthio.

Polyhalogenalkyloxy-Substituenten ($R^1$) können solche mit 1 bis 4 Kohlenstoffatomen sein, die als Halogenatome Fluor, Chlor oder Brom tragen, wobei die Halogenatome gleich oder verschieden sein können. Beispielsweise seien Trifluormethyloxy, Trichlormethyloxy, Tribrommethyloxy, Difluorchlormethyloxy, Difluorbrommethyloxy, Fluordichlormethyloxy, Fluordibrommethyloxy, Dibromchlormethyloxy, Bromdichlormethyloxy, Pentafluoräthyloxy, Pentachloräthyloxy, Pentabromäthyloxy, $\alpha$-Difluor-$\beta$-fluor-$\beta$-chlor-äthyloxy, die gleichartig oder verschiedenartig halogenierten Propyloxyreste oder die gleichartig oder verschiedenartig halogenierte Butyloxyreste gennant. Bevorzugt sind die Polyhalogenalkyloxy-Substituierten mit 1 bis 2 Kohlenstoffatomen, ganz bevorzugt sind die gleichartig oder verschiedenartig halogeniereten Methoxy-Substituenten.

Polyhalogenalkylthio-Substituenten ($R^1$) Können solche sein, wie sie als Polyhalogenalkyloxy-Substituenten gennant sind, wobei jedoch anstelle des Sauerstoffatoms ein Schwefelatom tritt. Sie können 1 bis 4 Kohlenstoffatome, bevorzugt 1 bis 2 Kohlenstoffatomen oder ganz besonders bevorzugt 1 Kohlenstoffatom haben.

Die Aryl-, Aralkyl-, Aryloxy- oder Arylthio-Substituenten ($R^1$) können in ihrem aromatischen Teil durch Halogen, beispielsweise Fluor, Chlor oder Brom, $C_1$—$C_4$-Alkyl, beispielsweise Methyl, Äthyl, Propyl oder Butyl, $C_1$—$C_4$-Polyhalogenalkyl, Polyhalogenalkyloxy oder Polyhalogenalkylthio, beispielsweise gleichartig oder verschiedenartig halogeniertes Methyl, Äthyl, Propyl oder Butyl, beziehungsweise Methoxy, Äthoxy, Propyloxy, Butyloxy, Methylthio, Äthylthio, Propylthio oder Butylthio oder durch Nitro, Chlorcarbonyl oder Chlorsulfonyl substituiert sein.

Die Alkylsubstituenten ($R^1$ bis $R^5$) können ihrerseits durch Halogenalkylgruppen substituiert sein.

Zwei benachbarte Reste $R^1$ bis $R^5$ können weiterhin gemeinsam einen 3- bis 5-gliedrigen Alkylenrest, beispielsweise Trimethylen, Tetramethylen oder Pentamethylen, bilden.

Als Beispiele für Verbindungen der Formel (I) seien genannt:

Benzol, Fluorbenzol, Chlorbenzol, Brombenzol, Toluol, 2-Chlortoluol, 3-Chlortoluol, 4-Chlortouol, 2,3-Dichlortoluol, 2,4-Dichlortoluol, 2,5-Dichlortoluol, 2,6-Dichlortoluol, 3,4-Dichlortoluol, 3,5-Dichlortoluol, die isomeren Difluor- bzw. Dibrom-toluole, die drei isomeren Xylole, 1-Chlor-2,3-dimethylbenzol, 1-Chlor-2,4-dimethylbenzol, 1-Chlor-3,4-dimethylbenzol, 1-Chlor-3,5-dimethylbenzol, Äthylbenzol, Isopropylbenzol, 2-Chlor-äthylbenzol, 3- und 4-Chlor-äthylbenzol, Mesitylen, Chlormesitylen, Brommesitylen, Durol, Chlordurol, Bromdurol, alle isomeren Dichlor-dimethylbenzole, Diphenyl, Alkyl- oder Halogen-substituierte Diphenyle, Alkyl- oder Halogen-substituierte Diphenyläther, Tetralin, Chlortetralin, Diphenylmethan, Pentafluoräthyl-phenyl-äther, Pentachloräthyl-phenyl-äther, Pentabromäthyl-phenyl-äther oder $\alpha$-Difluor-$\beta$-fluor-$\beta$-chlor-äthyl-phenyl-äther.

Das erfindungsgemäße Verfahren wird bevorzugt durch Umsetzung einer aromatischen Verbindung der Formel (II)

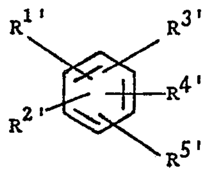

(II)

in der

$R^{1'}$ Wasserstoff, Alkyl, Phenyl, Phenyloxy oder Phenylthio bedeutet,

$R^{2'}$, $R^{3'}$ und $R^{4'}$ gleich oder verschieden sind und für Wasserstoff, Halogen oder Methyl stehen und

$R^{5'}$ Wasserstoff oder Alkyl bedeutet,

durchgeführt.

Das erfindungsgemäße Verfahren wird besonders bevorzugt durch Umsetzung einer aromatischen Verbindung der Formel (III)

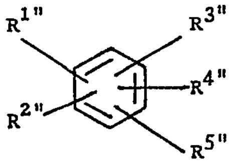

(III)

in der

$R^{1''}$ Wasserstoff, Methyl, Phenyloxy oder Phenylthio bedeutet,

$R^{2''}$, $R^{3''}$ und $R^{4''}$ gleich oder verschieden sind und für Wasserstoff, Halogen oder Methyl stehen und

$R^{5''}$ Wasserstoff oder Methyl ist

durchgeführt.

Für die quantitative Umsetzung der aromatischen Verbindung (I) sind mindestens 1 Mol Tetrachlorkohlenstoff und 3 Mol Fluorwasserstoff notwendig. Die einzelnen Rekationspartner können aber auch im Unterschuß oder im Überschuß eingesetzt werden. Im allgemeinen ist es jedoch sinnvoll, Tetrachlorkohlenstoff oder wasserfreien Fluorwasserstoff oder beide Reaktionspartner in Überschuß anzuwenden, und sie damit als Lösungs- und Verdünnungmittel einzusetzen. Die Reihenfolge der Zugabe der einzelnen Reaktionspartner ist für die Durchführung des erfindungsgemäßen Verfahrens ohne Bedeutung.

Die Reaktion des erfindungsgemäßen Verfahrens wird im Temperaturbereich von 50 bis 140°C durchgeführt. Bevorzugt arbeitet man im Temperaturbereich von 60 bis 130°C, besonders bevorzugt ist der Temperaturbereich von 80 bis 120°C.

Zur Durchführung des erfindungsgemäßen Verfahrens in der flüssigen Phase ist die Anwendung eines Uberdruckes notwendig. Dabei ist mindestens ein Druck notwendig, der dem Siedepunkt des Fluorwasserstoffs bei der gewählten Arbeitstemperatur entspricht. Im allgemeinen ist ein Druck von 5 bis 30 bar geeignet. Dieser Druck kann beispielsweise durch den Eigendruck der eingesetzten und der enstehenden Reaktionspartner erreicht werden. Es ist jedoch auch möglich, den gewählten Druck durch komprimierte atmosphärische Luft oder durch ein komprimiertes Inertgas, beispielsweise Stickstoff, einzustellen.

Die Reaktion des erfindungsgemäßen Verfahrens soll am Beispiel der Umsetzung von 1,4-Dichlor-2,5-dimethylbenzol mit Tetrachlorkohlenstoff und Fluorwasserstoff zu 2,5-Dichlor-3,6-dimethyl-benzotrifluorid durch die folgende Formelgleichung erläutert werden:

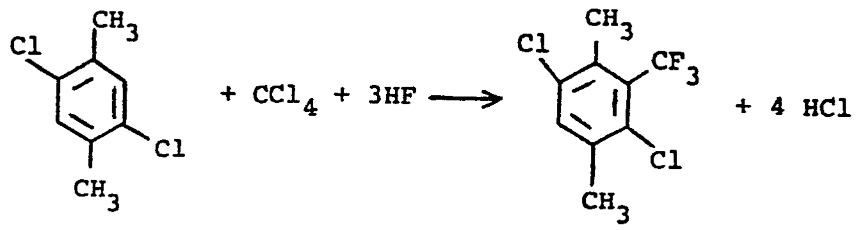

Das erfindungsgemäße Verfahren kann beispielsweise wie folgt durchgeführt werden:

In einem Autoklaven aus rostfreiem Stahl wird wasserfreier flüssiger Fluorwasserstoff unter Kühlung vorgelegt und eine Lösung der aromatischen Verbindung (I) in Tetrachlorkohlenstoff zugegeben. Anschließend wird mit Stickstoff ein Druck von etwa 3 bar eingestellt, und der Inhalt des Autoklaven wird unter Rühren auf die gewünschte Reaktionstemperatur gebracht. Nach kurzer Zeit zeigt sich der Beginn der Reaktion am Ansteigen des Druckes durch den entstehenden Chlorwasserstoff. Dieser Chlorwasserstoff wird über einen mit Sole gekühlten Rückflußkühler mit Regelventil kontinuierlich oder diskontinuierlich enspannt. Nach Abschluß der Reaktion werden der überschüssige Fluorwasserstoff und überschüssige Tetrachlorkohlenstoff abdestilliert und das Reaktionsprodukt, beispielsweise durch fraktionierte Destillation, isoliert.

Während die technische Synthese von Benzotrifluorid über die Stufen der Chlorierung der Methylgruppe und der nachfolgenden Fluorierung der Trichlormethylgruppe durchgeführt wird und eine Laboratoriumsdarstellung substituierter Benzotrifluoride gegebenenfalls nur durch vielstufige und möglicherweise verlustreiche Synthesen möglich ist, erlaubt das erfindungsgemäße Verfahren die Herstellung von aromatischen Trifluormethylverbindungen in nur einen Reaktionsschritt.

Es ist überraschend, daß das erfindungsgemäße Verfahren glatt und in guter Ausbeute die erfindungsgemäßen Endprodukte, liefert, da erwartet werden konnte, daß aromatische Verbindungen in Gegenwart einer Chloralkylverbindung wie Tetrachlorkohlenstoff und eines Friedel-Crafts-Katalysators weitergehende, möglicherweise unkontrollierbare Kondensations- oder Verharzungs-Reaktionen eingehen würde.

So ist beispielsweise bekannt, daß man aus Benzol und Tetrachlorkohlenstoff in Gegenwart von Aluminiumchlorid als Friedel-Crafts-Katalysator Tritylchlorid erhält (Oganikum, S. 302, 8. Aufl. VEB-Verlag, Berlin 1868). An gleicher Stelle wird auch über die Mehrfach-Alkylierung durch mehrfach halogenierte Alkane berichtet, so daß die selektive Einführung von $CF_3$-Gruppen in Aromaten durch das erfindungsgemäße Verfahren überraschend ist.

Die nach dem erfindungsgemäßen Verfahren herstellbaren Benzotrifluoride sind wichtige Zwischenprodukte für Farbstoffe, Insektizide und Herbizide. So erhält man beispielsweise aus 2-Methyl-4-chlorbenzotrifluorid durch Nitrierung das 2-Methyl-3,5-dinitro-4-chlor-benzotrifluorid, das sich mit sekundären Aminen zu N,N-disubstituierten 2,6-Dinitro-3-methyl-4-trifluormethylanilinen umsetzen läßt, die als Wachstumsregulatoren und Herbizide wirksam sind (DE—OS 2 232 263 und DE—OS 2 241 408).

Die Darstellung weiterer wirksamer Insektizide und Herbizide, aber auch weiterer Farbstoffe, die aromatische Trifluormethylverbindungen als Zwischenprodukte erfordern, scheiterte bisher an der mangelnden Zugänglichkeit geeigneter aromatischer Trifluormethylverbindungen.

Alle Siedepunktsangaben unter vermindertem Druck beziehen sich auf Druckangaben in mbar.

## Beispiel 1

In einem Autoklaven aus VA mit Rührer, Rückflußkühler und Druckregelventil werden 1500 g wasserfreier Fluorwasserstoff, 860 g 3-Chlortoluol und 2500 g Tetrachlorkohlenstoff bei 10°C vorgelegt. Dann werden 3 bar Stickstoff aufgedrückt und unter Rühren für 12 Stunden auf 102 bis 104°C erhitzt. Der entstehende Chlorwasserstoff wird bei 15,5 bar kontinuierlich entspannt. Nach Ende der Reaktion wird der überschüssige Fluorwasserstoff und der Tetrachlorkohlenstoff abdestilliert. Das Reaktionsprodukt wird über eine Kolonne destilliert und man erhält 290 g 2-Methyl-4-chlorbenzotrifluorid, $Kp_{66}$: 83—4°C, 85 g Zwischenlauf und 922 g 4-Methyl-2-chlorbenzotrifluorid, $Kp_{66}$: 91°C $n_D^{20}$: 1.4600.

## Beispiel 2

In einem VA-Autoklaven werden 900 ml Fluorwasserstoff, 1200 ml Tetrachlorkohlenstoff und 300 g 2-Chlortoluol bei 0°C vorgelegt. Unter Rühren wird für 6 Stunden bei 107 bis 110°C gehalten und der entstehende Chlorwasserstoff in dem Maße entspannt, wie sich etwa 25 bar Druck einhalten lassen. Nach Ende der Reaktionszeit wird abgekühlt und der Reaktionsinhalt durch Destillation aufgearbeitet. Man erhält 395 g einer Mischung, die nach gaschromatographischer Analyse zu 25 % aus 2-Chlortoluol, 15,7 % 4-Methyl-3-chlor-benzotrifluorid ($Kp_{66}$: 80—1°C) und 59,1 % 3-Methyl-2-chlorbenzotrifluorid ($Kp_{66}$: 93—4°C) besteht. Die Reaktionsprodukte werden durch fraktionierte Destillation getrennt.

Beispiel 3

Eine Mischung aus 1500 ml Fluorwasserstoff, 400 g Mesitylen und 1500 ml Tetrachlorkohlenstoff werden für 18 Stunden auf 92—3°C erhitzt und der Chlorwasserstoff bei 11,5 bar entspannt. Dann wird der Fluorwasserstoff abdestilliert und der Rückstand fraktioniert. Man erhält 385 g 2,4,6-Trimethylbenzotrifluorid, $Kp_{20}$: 55—8°C; $n_D^{20}$: 1.4550, was einer Ausbeute von 61 % der Theorie entspricht.

Beispiel 4

Bei 10°C werden in einem Autoklaven 300 ml Fluorwasserstoff, 900 ml Tetrachlorkohlenstoff und 100 g Toluol vorgelegt und anschließend unter Rühren für 10 Stunden auf 106°C erhitzt und der Chlorwasserstoff bei 16,4 bar entspannt. Nach der Aufarbeitung analog Beispiel 1 fallen 102 g Reaktionsprodukt an, die zu 10,6 % aus 3-Methylbenzotrifluorid (Kp: 131°C; $n_D^{20}$: 1.4258, 48 % aus 2-Methylbenzotrifluorid (Kp: 128 bis 129°C; $n_D^{20}$: 1.4322) und 41,4 % aus 4-Methylbenzotrifluorid (Kp: 134°C; $n_D^{20}$ 1.4255) bestehen. Die drei Methylbenzotrifluoride können durch Destillation über eine Kolonne getrennt werden.

Beispiel 5

Ähnlich wie in Beispiel 1 werden 500 ml Fluorwasserstoff, 600 ml Tetrachlorkohlenstoff und 140 g 2,5-Dimethylchlorbenzol bei 101—3°C in 10 Stunden zur Reaktion gebracht. Durch Destillation erhält man 191 g 2,5-Dimethyl-4-chlorbenzotrifluorid, $Kp_{20}$: 81—83°C; $n_D^{20}$: 1,4715.

Beispiel 6

Analog Beispiel 1 werden 400 ml Fluorwasserstoff, 500 ml Tetrachlorkohlenstoff und 100 g 2,5-Dichlor-p-xylol für 10 Stunden bei 100°C und einem Druck von 14,5 bar umgesetzt. Man erhält 90 g 2,5-Dimethyl-3,6-dichlorbenzotrifluorid, F: 62 bis 63°C.

Beispiel 7

500 ml Fluorwasserstoff, 600 ml Tetrachlorkohlenstoff und 140 g 3,4-Dichlortoluol werden bei 110 bis 115°C und 18,5 bis 19,5 bar Druck umgesetzt. Man erhält 151 g Stubstanz, in denen neben 79,1 Gew.-% 3,4-Dichlortoluol 20,2 Gew.-% 2-Methyl-4,5-dichlor-benzotrifluorid enthalten sind, was 74 % der theoretischen Ausbeute, bezogen auf umgesetztes 3,4-Dichlortoluol, bedeutet.

Beispiel 8

Setzt man 400 ml Fluorwasserstoff, 170 g Diphenyläther und 400 ml Tetrachlorkohlenstoff für 6 Stunden bei 112 bis 115°C um, so erhält man 20 g 4-Trifluormethyldiphenyläther, $Kp_{18}$: 125—127°C.

Beispiel 9

200 g Pentamethylbenzol werden zusammen mit 750 ml Tetrachlorkohlenstoff und 500 g wasserfreiem Fluorwasserstoff für 10 Stunden auf 80 bis 82°C erhitzt und der entstehende Chlorwasserstoff bei 14 bar entspannt. Durch destillative Aufarbeitung des Reaktionsgemisches erhält man neben 35 g Ausgangsmaterial 112 g Pentamethylbenzotrifluorid, $Kp_{18}$: 108 bis 112°C.

Beispiel 10

200 g Brombenzol, 500 ml Tetrachlorkohlenstoff und 500 g wasserfreier Fluorwasserstoff werden analog Beispiel 1 in 10 Stunden bei 108 bis 110°C und 18 bar umgesetzt. Man erhält 212 g Reaktionsprodukt im Siedebereich $Kp_{49}$: 64 bis 74°C, das zu 61,1 Gew.-% aus Brombenzol, 14,3 Gew.-% 4-Brombenzotrifluorid und 23,5 Gew.-% 2-Brombenzotrifluorid besteht. Die Gewichtsverhältnisse im Reaktionsprodukt wurden durch gaschromatographische Analyse bestimmt. Das Gemisch läßt sich durch Destillation über eine Kolonne auftrennen. Die Ausbeute der beiden isomeren Brombenzotrifluoride beträgt 85 % der theoretischen Ausbeute, bezogen auf umgesetztes Brombenzol.

Beispiel 11

Analog Beispiel 1 werden 500 g wasserfreier Fluorwasserstoff, 600 ml Tetrachlorkohlenstoff und 110 g 3-Fluortoluol bei 105°C und 16,7 bar in 8 Stunden umgesetzt. Man erhält 132 g eines Reaktionsgemisches, das nach gaschromatographischer Analyse neben 33,7 Gew.-% 3-Fluortoluol noch 64,4 Gew.-% 3-Methyl-4-fluor-benzotrifluorid enthält. Die Ausbeute an 3-Methyl-4-fluorbenzotrifluorid beträgt 87 % der theoretischen Ausbeute, bezogen auf verbrauchtes 3-Fluortoluol.

Beispiel 12

Nach der Arbeitsweise von Beispiel 1 werden 400 g wasserfreier Fluorwasserstoff, 100 g 4-Nitro-2'-methyl-diphenyläther und 700 ml Tetrachlorkohlenstoff bei 110°C und einem Druck von 18 bar für 6 Stunden zur Reaktion gebracht. Durch Destillation erhält man 118 g 4-Nitro-2'-trifluormethyl-6'-methyl-diphenyläther, $Kp_{0.8}$: 150 bis 155°C, $n_D^{20}$: 1.5762.

Beispiel 13

Wie in Beispiel 1 wird eine Mischung aus 400 g wasserfreiem Fluorwasserstoff, 120 g 3,3'-Dimethyl-diphenyläther und 600 ml Tetrachlorkohlenstoff für 6 Stunden auf 85 bis 88°C bei einem Druck von 12 bar erhitzt und der entstehende Chlorwasserstoff kontinuierlich entspannt. Nach der Aufarbeitung durch Destillation erhält man 53 g 2-Trifluormethyl-5,3'-dimethyl-diphenyläther, $Kp_{0,1}$: 98 bis 100°C.

Beispiel 14

In einem Autoklaven aus VA-Material (rostfreier Stahl) werden 250 ml wasserfreier Fluorwasserstoff bei 10°C vorgelegt und eine Mischung aus 50 g Benzol in 300 ml Tetrachlorkohlenstoff zugegeben. Dann werden 3 bar Stickstoff aufgedrückt, und es wird für 5 Stunden auf 100°C erhitzt und der Chlorwasserstoff kontinuierlich bei einem Druck von 15 bar entspannt. Aus dem erhaltenen Reaktionsgemisch wird der überschüssige Fluorwasserstoff bei einer Temperatur von bis zu 50°C abdestilliert. Sodann wird die verbliebene Lösung mit Wasser gewaschen und mit Natriumsulfat getrocknet. Die erhaltene Lösung (387 g) wird durch Gaschromatographie analysiert. Sie enthält 84,4 Gew.-% Tetrachlorkohlenstoff und 13,43 Gew.-% Benzotrifluorid, was einer Ausbeute von 52 g an Benzotrifluorid entspricht.

Beispiel 15

500 ml wasserfreier Fluorwasserstoff werden mit 700 ml Tetrachlorkohlenstoff und 100 g 2,4-Dimethyl-phenyl-trifluormethylthioäther analog Beispiel 1 für 9 Stunden bei 105°C und einem Druck von 15,3 bar zur Reaktion gebracht. Nach der Aufarbeitung durch Destillation werden 106 g Reaktionsgemisch mit einem $Kp_{14}$ von 74 bis 79°C und einem $n_D^{20}$ von 1,4700 erhalten, das nach gaschromatographischer Analyse 79,5 Gew.-% Ausgangsmaterial und 20,1 Gew.-% Trifluormethyl-substituierten 2,4-Dimethyl-phenyl-trifluormethylthioäther enthält.

Beispiel 16

Analog Beispiel 1 wird eine Mischung aus 500 ml wasserfreiem Fluorwasserstoff, 100 g 4-Diphenyl-carbonsäurechlorid und 500 ml Tetrachlorkohlenstoff für 6 Stunden bei einem Druck von 19,5 bar auf 110°C erhitzt. Die Aufarbeitung durch Destillation ergibt 74 g 4'-Trifluormethyl-4-diphenyl-carbonsäurefluorid, $Kp_{0,3}$: 122 bis 124°C, Fp.: 93 bis 95°C.

Beispiele 17 bis 20

Die in der folgenden Tabelle enthaltenen Beispiele wurden analog Beispiel 1 durchgeführt, jedoch unter Ersatz des 3-Chlortoluols durch die in der Tabelle angegebenen Ausgangsstoffe.

Tabelle zu Beispiele 17 bis 20

| Nr. | Ausgangsstoff | Reaktionsprodukt | Physikalische Daten |
|---|---|---|---|
| 17 | 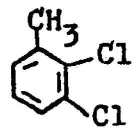 | 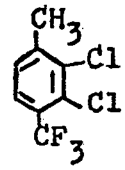 | $Kp_{67}$: 123 °C<br>$n_D^{20}$ : 1.4960 |
| 18 | 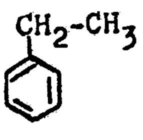 | 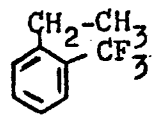 + 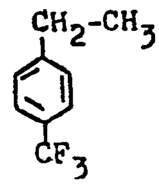<br>Hauptprodukt | Hauptprodukt:<br>$Kp_{197}$: 88–90 °C<br>$n_D^{20}$ : 1.4508 |
| 19 | 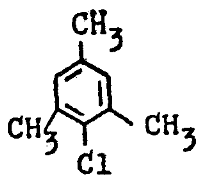 | 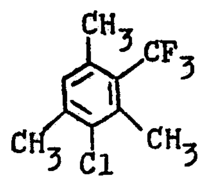 | $Kp_{97}$: 136 °C<br>$n_D^{20}$ : 1.4868 |
| 20 | 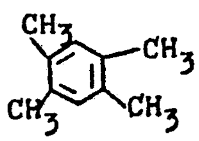 | 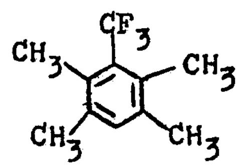 | $Kp_{18}$: 75–76 °C |

Beispiel 21

Wie in Beispiel 1 beschrieben werden 400 g wasserfreier Fluorwasserstoff, 150 g 4-Nitro-3'-methyl-diphenylether und 800 ml Tetrachlorkohlenstoff für 6 Stunden bei 110°C und 18 bar zur Reaktion gebracht. Nach der Aufarbeitung durch Destillation erhält man 140 g eines Reaktionsgemisches vom $Kp_{0.1}$: 110 bis 130°C, aus dem 4-Nitro-4'-trifluormethyl-3'-methyl-diphenylether auskristallisiert, Fp.: 66 bis 68°C.

Beispiel 22

Aus 100 g 4-Nitrophenyl, 500 ml Tetrachlorkohlenstoff und 400 ml wasserfreiem Fluorwasserstoff werden nach 8 Stunden bei 110°C und 117 bis 19 bar analog Beispiel 1 108 g 4-Trifluormethyl-4'-nitrobiphenyl erhalten, Fp.: 112 bis 114°C nach Umkristallisation aus Methanol.

**Patentansprüche**

1. Verfahren zur Herstellung von aromatischen Trifluormethylverbindungen, dadurch gekennzeichnet, daß man eine aromatische Verbindung der Formel (I)

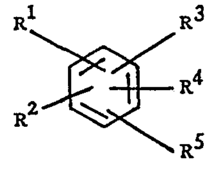

in der

$R^1$ Wasserstoff, Alkyl, Aryl, Aralkyl, Aryloxy, Arylthio, Polyhalogenalkyloxy oder Polyhalogenalkylthio bedeutet, wobei die aromatischen Substituenten $R^1$ ihrerseits durch Halogen, Alkyl, Polyhalogenalkyl, Polyhalogenalkyloxy, Polyhalogenalkylthio, Nitro, Chlorcarbonyl oder Chlorsulfonyl substituiert sein können,

$R^2$, $R^3$, $R^4$ und $R^5$ gleich oder verschieden sind und Wasserstoff, Halogen oder Alkyl bedeuten, sowie zwei benachbarte Reste aus der Gruppe $R^1$ bis $R^5$ gemeinsam einen 3- bis 5-gliedrigen Alkylenrest bilden können,

wobei die Substituenten $R^1$ bis $R^5$, wenn sie Alkyl bedeuten, durch Halogenalkyl substituiert sein können, mit Tetrachlorkohlenstoff und Fluorwasserstoff im Temperaturbereicht von 50°C bis 140°C umsetzt.

2. Verfahren nach Anspruch 1, dadurch gekennzeichnet, daß man eine aromatische Verbindung der Formel (II)

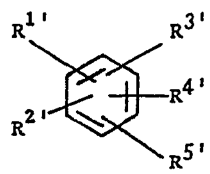

umsetzt,
in der

$R^{1'}$ Wasserstoff, Alkyl, Phenyl, Phenyloxy oder Phenylthio bedeutet,

$R^{2'}$, $R^{3'}$ und $R^{4'}$ gleich oder verschieden sind und für Wasserstoff, Halogen oder Methyl stehen und

$R^{5'}$ Wasserstoff oder Alkyl bedeutet.

3. Verfahren nach Anspruch 1, dadurch gekennzeichnet, daß man eine aromatische Verbindung der Formel (III)

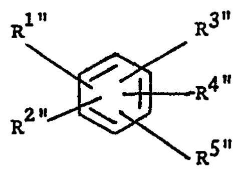

umsetzt,
in der

$R^{1''}$ Wasserstoff, Methyl, Phenyloxy oder Phenylthio bedeutet,

$R^{2''}$, $R^{3''}$ und $R^{4''}$ gleich oder verschieden sind und für Wasserstoff, Halogen oder Methyl stehen und

$R^{5''}$ Wasserstoff oder Methyl ist.

4. Verfahren nach Anspruch 1 bis 3, dadurch gekennzeichnet, daß man mindestens 1 Mol Tetrachlorkohlenstoff und mindestens 3 Mol Fluorwasserstoff pro Mol aromatische Verbindung umsetzt.

5. Verfahren nach Anspruch 1 bis 4, dadurch gekennzeichnet, daß man die Umsetzung bei 60—130°C durchführt.

6. Verfahren nach Anspruch 1 bis 5, dadurch gekennzeichnet, daß man die Umsetzung in flüssiger Phase bei einem Druck von 5 bis 30 bar durchführt.

7. Verfahren nach Anspruch 1 bis 6, dadurch gekennzeichnet, daß man die Umsetzung unter dem Eigendruck der Reaktionspartner, der sich bei der Reaktionstemperatur einstellt, durchführt.

**Claims**

1. Process for the preparation of aromatic trifluormethyl compounds, characterised in that an aromatic compound of the formula (I)

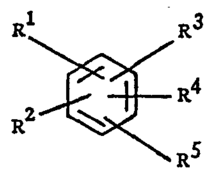

in which

$R^1$ denotes hydrogen, alkyl, aryl, aralkyl, aryloxy, arylthio, polyhalogenoalkyloxy or polyhalogenoalkylthio, and the aromatic substituents $R^1$ can in turn be substituted by halogen, alkyl, polyhalogenoalkyl, polyhalogenoalkyloxy, polyhalogenoalkylthio, nitro, chlorocarbonyl or chlorosulphonyl,

$R^2$, $R^3$, $R^4$ and $R^5$ are identical or different and denote hydrogen, halogen or alkyl,

and two adjacent radicals from the group $R^1$ to $R^5$ can conjointly form a 3-membered to 5-membered alkylene radical,

and the substituents $R^1$ to $R^5$, when they denote alkyl, can be substituted by halogenoalkyl, is reacted with carbon tetrachloride and hydrogen fluoride in the temperature range of 50°C to 140°C.

2. Process according to Claim 1, characterised in that an aromatic compound of the formula (II)

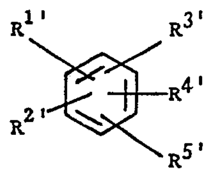

is reacted, in which

$R^{1'}$ denotes hydrogen, alkyl, phenyl, phenyloxy or phenylthio,
$R^{2'}$, $R^{3'}$ and $R^{4'}$ are identical or different and denote hydrogen, halogen or methyl and
$R^{5'}$ denotes hydrogen or alkyl.

3. Process according to Claim 1, characterised in that an aromatic compound of the formula (III)

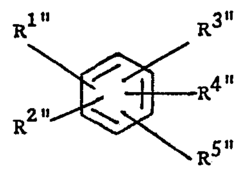

is reacted, in which

$R^{1''}$ denotes hydrogen, methyl, phenyloxy or phenylthio,
$R^{2''}$, $R^{3''}$ and $R^{4''}$ are identical or different and denote hydrogen, halogen or methyl and
$R^{5''}$ is hydrogen or methyl.

4. Process according to Claim 1 to 3, characterised in that at least 1 mol of carbon tetrachloride and at least 3 mols of hydrogen fluoride are reacted per mol of aromatic compound.

5. Process according to Claim 1 to 4, characterised in that the reaction is carried out at 60—130°C.

6. Process according to Claim 1 to 5, characterised in that the reaction is carried out in the liquid phase at a pressure of 5 to 30 bars.

7. Process according to Claim 1 to 6, characterised in that the reaction is carried out under the autogenous pressure of the reactants which forms at the reaction temperature.

**Revendications**

1. Procédé de préparation de composés trifluorométhyliques aromatiques, caractérisé en ce que, à une température se situant dans l'intervalle allent de 50 à 140°C, on fait réagir, avec du tétrachlorure de carbone et de l'acide fluorhydrique, un composé aromatique répondant à la formule (I):

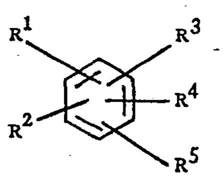

dans laquelle

$R^1$ représente un atome d'hydrogène, un groupe alkyle, un groupe aryle, un groupe aralkyle, un groupe aryloxy, un groupe arylthio, un groupe polyhalogénalkyloxy. ou un groupe polyhalogénnalkyloxy ou un groupe polyhalogénalkylthio, les substituants aromatiques $R^1$ pouvant, à leur tour, être substitués par un atome d'halogène, un groupe alkyle, un groupe polyhalogénalkyle, un groupe polyhalogénalkyloxy, un groupe polyhalogénalkylthio, un groupe nitro, un groupe chlorocarbonyle ou un groupe chlorosulfonyl,

$R^2$, $R^3$, $R^4$ et $R^5$ sont identiques ou différents et représentent chacun un atome d'hydrogène, un atome d'halogène ou un groupe alkyle,

deux radicaux voisins choisis parmi les groupes $R^1$ à $R^5$ pouvant former, ensemble, un groupe alkylène de 3 à 5 termes,

les substituants $R^1$ à $R^5$ pouvant, lorsqu'ils représentent des groupes alkyle, être substitués par un groupe halogénalkyle.

2. Procédé suivant la revendication 1, caractérisé en ce qu'on fait réagir un composé aromatique de formule (II):

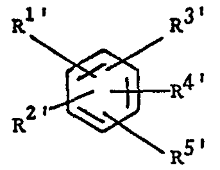

dans laquelle

$R^{1'}$ représente un atome d'hydrogène, un groupe alkyle, un groupe phényle, un groupe phényloxy ou un groupe phénylthio,

$R^{2'}$, $R^{3'}$ et $R^{4'}$ sont identiques ou différents et représentent chacun un atome d'hydrogène, un atome d'halogène ou un groupe méthyle, et

$R^{5'}$ représente un atome d'hydrogène ou un groupe alkyle.

3. Procédé suivant la revendication 1, caractérisé en ce qu'on fait réagir un composé aromatique répondant à la formule (III):

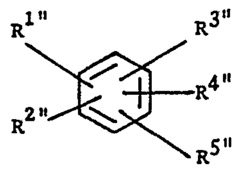

dans laquelle

$R^{1''}$ représente un atome d'hydrogène, un groupe méthyle, un groupe phényloxy ou un groupe phénylthio,

$R^{2''}$, $R^{3''}$ et $R^{4''}$ sont identiques ou différents et représentent chacun un atome d'hydrogène, un atome d'halogène ou un groupe méthyle, et

$R^{5''}$ représente un atome d'hydrogène ou un groupe méthyle.

4. Procédé suivant les revendications 1 à 3, caractérisé en ce qu'on fait réagir au moins 1 mole de tétrachlorure de carbone et au moins 3 moles d'acide fluorhydrique par mole du composé aromatique.

5. Procédé suivant les revendications 1 à 4, caractérisé en ce qu'on effectue la réaction à une température de 60 à 130°C.

6. Procédé suivant les revendications 1 à 5, caractérisé en ce qu'on effectue la réaction en phase liquide sous une pression de 5 à 30 bars.

7. Procédé suivant les revendications 1 à 6, caractérisé en ce qu'on effectue la réaction sous la pression propre des partenaires réactionnels, cette pression s'établissant à la température de la réaction.